Europäisches Patentamt

(19) European Patent Office

Office européen des brevets

(11) Numéro de publication: **0 204 601**
**B1**

(12) ## FASCICULE DE BREVET EUROPEEN

(45) Date de publication du fascicule du brevet:
24.01.90

(21) Numéro de dépôt: **86401037.6**

(22) Date de dépôt: **15.05.86**

(51) Int. Cl.⁴: **A61K 33/12**

(54) **Compositions anti-diarrhéiques.**

(30) Priorité: **15.05.85 GB 8512345**

(43) Date de publication de la demande:
**10.12.86 Bulletin 86/50**

(45) Mention de la délivrance du brevet:
**24.01.90 Bulletin 90/4**

(84) Etats contractants désignés:
**BE CH DE IT LI LU NL SE**

(56) Documents cités:
**CH-A- 643 144**
**FR-A- 2 223 029**
**FR-A- 2 346 017**

(73) Titulaire: **SOCIETE DE CONSEILS DE RECHERCHES ET D'APPLICATIONS SCIENTIFIQUES (S.C.R.A.S.),**
**51/53 rue du Docteur Blanche, F-75016 Paris(FR)**

(72) Inventeur: **Bhargava, Hridaya, 21 Sherwood Circle, Sharon Massachusetts(US)**
Inventeur: **Jutteau, Jacques, 19 rue du Calvaire, F-92210 Saint-Cloud(FR)**

(74) Mandataire: **Lachassagne, Jacques, Boite Postale 07, F-78430 Louveciennes(FR)**

**Description**

La présente invention concerne de nouvelles compositions thérapeutiques à action anti-diarrhéique.

Il est connu de traiter la diarrhée soit par l'administration d'une substance absorbante, absorbant les bactéries pathogènes, les enzymes digestifs, les toxines et les éléments nutritifs de l'appareil gastro-intestinal ; soit par l'administration, par voie orale, d'une solution de complément contenant un électrolyte et du sucre, ainsi que cela est recommandé par l'Organisation Mondiale de la Santé ; un tel liquide de complément contient, par litre, 20 g de glucose, 90 mEq de sodium, 30 mEq de bicarbonate et 20 mEq de potassium (Chatterjee et al. (1978) Arch. Dis. Child 53, 284).

La présente invention concerne une nouvelle composition à action anti-diarrhéique contenant des quantités suffisantes pour réduire la diarrhée d'une argile à base de silicate double d'aluminium et de magnésium, thermiquement activée et finement pulvérisée, comme substance susceptible d'absorber les bactéries intestinales pathogènes, d'un sel de sodium, d'un sel de potassium et de sucre.

La substance absorbante est, de préférence, également susceptible d'absorber les virus associés à la diarrhée, les toxines intestinales ainsi que les gaz. On connaît un certain nombre de substances absorbantes convenant à cet usage. Il existe, en particulier, une argile connue sous sa forme non activée sous le nom de smectite et répondant à la formule Si*Al$O@)OH$. On connaît également d'autres matériaux argileux comme, par exemple, l'attapulgite de mormoiron que l'on appellera ATTA par mesure d'abréviation et qui est également susceptible d'être activée pour constituer une substance absorbante, agissant contre la diarrhée.

La substance absorbante est apportée en quantité suffisante pour pouvoir agir dans le traitement de la diarrhée et est conditionnée dans un paquet dont le contenu est destiné à permettre la reconstitution du médicament à administrer en lui ajoutant 200 ml d'eau. La substance absorbante se trouve, alors, en quantité telle que sa concentration dans la forme d'administration est comprise entre 2,5 et 15 g/l.

Les ingrédients solides permettant de réaliser la solution de complément indiquée plus haut comprennent un sel de potassium, un sel de sodium et un sucre.

De préférence, ces deux sels sont des chlorures de potassium et de sodium tandis que le sucre est du glucose ou du dextrose. La solution contient, de préférence, un bicarbonate tel que le bicarbonate de sodium.

Comme en général les ingrédients solides de la solution de complément sont fournis sous une forme déshydratée et pulvérulente, il est préférable de prendre des mesures apropriées pour empêcher que l'humidité amène l'agglomération de la poudre, ce qui aurait un inconvénient lors de la reconstitution de la solution de complément. Cela peut être obtenu en conditionnant le matériau en poudre dans un sachet étanche, comme par exemple un sachet en feuille d'aluminium, ou en ajoutant un agent déssechant, ou encore en traitant les ingrédients solides de complément de telle sorte que soit empêchée l'absorbtion ultérieure d'humidité, par exemple, par formation de cristaux ainsi que cela est décrit dans le brevet américain US-A 2 624 335. Les deux dernières solution présentent l'avantage de permettre l'utilisation d'un emballage à faible coût comme le papier paraffiné qui n'est pas complètement imperméable à l'humidité.

Les constituants de l'électrolyte et le sucre sont apportés en quantités suffisantes pour agir dans le traitement de la diarrhée. Les ingrédients solides permettant de reconstituer la solution sont, également, apportés en quantités telles que, lors de la reconstitution, ils se trouvent présents dans la forme d'administration dans la gamme de concentrations suivantes : glucose : 10 à 30 g/l ; sodium : 60 à 120 mEq/l ; bicarbonate : 10 à 50 mEq/l ; potassium : 10 à 30 mEq/l.

Dans une forme préférée de réalisation de l'invention, la composition contient également un agent de mise en suspension et d'homogénéisation pour disperser la substance absorbante dans l'eau ; en l'absence d'un tel agent, les argiles telles que la smectite auraient tendance à sédimenter dans le fond du conditionnement, ce qui rendrait difficile l'administration de la composition thérapeutique sous forme homogène. L'agent de mise en suspension peut être, par exemple, un hydrocolloide (comme la carboxyméthyle cellulose sodique, la gomme xanthique, l'hydroxypropylméthyle cellulose, le polyéthylène glycol, une dextrine, la gomme karaya, la gomme adragante, la gomme d'acacia, la gomme guar ou un polysaccharide), un polyalcool (tel que le glycérine, le propylène glycol ou le sorbitol) ou un surfactif (tel que le dioctylsulfossucinate, le polysorbate-40® ou le monooléate de sorbitan).

L'agent de mise en suspension est prévu en une quantité telle que, lorsque la composition est reconstituée, il se trouve présent à une concentration de 0,25 à 1 g/l, dans le cas des hydrocolloides naturels tels que la gomme xanthique, ou 1 à 5 g/l dans le cas des autres types de produits. Dans certains cas, on peut utiliser plus d'un agent de mise en suspension.

En plus des composants indiqués ci-dessus, on peut également rajouter des agents colorants, aromatisants et édulcorants.

Pour préparer la composition sèche destinée à être emballée et à permettre la reconstitution d'une préparation pharmaceutique, l'agent ou les agents de mise en suspension seront d'abord placés dans un mélangeur. On ajoute ensuite la substance absorbante et on mélange l'ensemble pendant 10 à 20 minutes. On ajoute enfin les sels et sucre, un par un, en continuant le mélange pendant 10 à 20 minutes de plus. Puis on conditionne la composition sèche dans des paquets formés soit en papier paraffiné, soit dans une matière synthétique ou en feuilles plastiques, chaque paquet contenant la quantité désirée pour reconstituer, par addition de 200 ml d'eau, la préparation pharmaceutique.

Ainsi que cela a été mentionné plus haut, on peut également prévoir un traitement des sels pour éviter la reprise d'humidité, selon la technique exposée dans le brevet américain US-A 2 642 335.

Posologie

Le nombre de paquets administrés quotidiennement est variable selon le cas et tient compte de l'état du sujet et de son poids corporel. Notamment pour un enfant de moins de 5 kg, la dose conseillée est d'un paquet par jour, réparti en 3 prises égales espacées de 8 heures. Pour un enfant pesant entre 5 et 10 kg, la posologie conseillée est de 1 à 2 paquets par jour et pour ceux de plus de 10 kg, elle est de 2 paquets par jour.

On donnera ci-dessous, à titre d'exemples, la composition de plusieurs formes de dosages, tous destinés à être additionnés de 200 ml d'eau pour constituer le médicament réellement administré. Les quantités dans les exemples sont exprimées en g par paquet. Dans les compositions 2, 3 et 4, plusieurs variantes sont proposées.

**Composition 1**

| Ingrédients | Grammes par paquet |
| --- | --- |
| Smectite | 1,0 |
| Chlorure de sodium* | 0,7 |
| Chlorure de potassium* | 0,3 |
| Bicarbonate de sodium* | 0,5 |
| Monohydrate de glucose* | 4,0 |

\* Equivalent à 18 mEq de sodium pour 200 ml, 4 mEq de potassium pour 200 ml, 6 mEq de bicarbonate pour 200 ml et 4,0 g de glucose pour 200 ml.

**Composition 2**

| | A | B | C | D |
| --- | --- | --- | --- | --- |
| Smectite | 1,0 | 1,0 | 1,0 | 1,0 |
| Chlorure de sodium | 0,7 | 0,7 | 0,7 | 0,7 |
| Chlorure de potassium | 0,3 | 0,3 | 0,3 | 0,3 |
| Bicarbonate de sodium | 0,5 | 0,5 | 0,5 | 0,5 |
| Monohydrate de glucose | 4,0 | 4,0 | 4,0 | 4,0 |
| Gomme xanthique | – | – | – | 0,05-0,2 |
| Hydroxypropylméthyle cellulose | – | – | 0,5-1,0 | – |
| Carboxyméthyle cellulose sodique | 0,2-1,0 | – | – | – |

Composition 3

| Ingrédients | A | B | C | D | E | F | G | H | I | J |
|---|---|---|---|---|---|---|---|---|---|---|
| Smectite | 1,0 | 1,0 | 1,0 | 1,0 | 1,0 | 1,0 | 1,0 | 1,0 | 1,0 | 1,0 |
| Chlorure de sodium | 0,7 | 0,7 | 0,7 | 0,7 | 0,7 | 0,7 | 0,7 | 0,7 | 0,7 | 0,7 |
| Chlorure de potassium | 0,3 | 0,3 | 0,3 | 0,3 | 0,3 | 0,3 | 0,3 | 0,3 | 0,3 | 0,3 |
| Bicarbonate de sodium | 0,5 | 0,5 | 0,5 | 0,5 | 0,5 | 0,5 | 0,5 | 0,5 | 0,5 | 0,5 |
| Monohydrate de glucose | 4,0 | 4,0 | 4,0 | 4,0 | 4,0 | 4,0 | 4,0 | 4,0 | 4,0 | 4,0 |
| Gomme karaya | 0,05 0,2 | — | — | — | — | — | — | 0,1 | — | 0,1 |
| Gomme guar | — | 0,05 2,0 | — | — | — | — | — | — | 1,0 | — |
| Acacia | — | — | 0,5 2,0 | — | 0,5 2,0 | — | 0,79 | — | — | 0,5 |
| Polyéthylène Glycol 6000 | — | — | — | 2,0 | — | — | 3,0 | — | — | — |
| Glycérine | 0,5 | 0,5 | 0,5 | — | — | — | — | — | 0,5 | — |
| Dioctylsulfosuccinate de sodium | 0,1 | 0,1 | 0,1 | — | — | — | — | — | 0,01 | — |
| Polysorbate-40® | — | — | — | — | 0,1 | — | — | 0,05 | — | — |
| Maltodextrine | — | — | — | — | — | 1,0 | 0,1 | — | — | 0,5 |

Composition 4

| Ingrédients | A | B | C | D | E | F | G | H | I | J |
|---|---|---|---|---|---|---|---|---|---|---|
| Smectite | 1,0 | 1,0 | 1,0 | 1,0 | 1,0 | 1,0 | 1,0 | 1,0 | 1,0 | 1,0 |
| Chlorure de sodium | 0,7 | 0,7 | 0,7 | 0,7 | 0,7 | 0,7 | 0,7 | 0,7 | 0,7 | 0,7 |
| Chlorure de potassium | 0,3 | 0,3 | 0,3 | 0,3 | 0,3 | 0,3 | 0,3 | 0,3 | 0,3 | 0,3 |
| Bicarbonate de sodium | 0,5 | 0,5 | 0,5 | 0,5 | 0,5 | 0,5 | 0,5 | 0,5 | 0,5 | 0,5 |
| Monohydrate de glucose | 4,0 | 4,0 | 4,0 | 4,0 | 4,0 | 4,0 | 4,0 | 4,0 | 4,0 | 4,0 |
| Gomme xanthique | 0,1 | 0,1 | — | — | 0,1 | 0,1 | 0,1 | — | — | — |
| Sodium CMC | 0,1 | — | 0,1 | 0,2 | — | 0,1 | — | — | — | |
| Glycérine | — | 0,1 | 0,1 | — | — | — | — | 0,5 | — | 0,5 |
| Polyéthylène glycol 6000 | — | — | — | 2,0 | — | — | — | 1,0 | 5,0 | — |
| Maltodextrine | — | — | — | — | 0,5 | 0,5 | 0,5 | — | 2,0 | 2,0 |
| Dioxide de silicium | — | — | — | — | 0,1 | — | 0,1 | — | — | — |
| Chlorure de calcium | — | — | — | — | — | 0,2 | — | — | 0,2 | 0,2 |
| Benzoate de sodium | 0,1 | — | — | 0,1 | 0,1 | 0,1 | 0,1 | — | 0,1 | 0,1 |
| Hydroxypropylméthyle cellulose | — | — | — | — | — | — | — | — | — | 0,75 |
| Gomme adragante | — | — | — | — | — | — | — | 0,5 | — | — |

Composition 5

| Ingrédients | Grammes par paquet |
|---|---|
| Citrate de sodium dihydraté | 0,60 |
| Chlorure de potassium | 0,30 |
| Chlorure de sodium | 0,66 |
| Monohydrate de dextrose | 4,00 |
| Gomme xanthique | 0,10 |
| Maltodextrine | 0,50 |
| Triphosphate de calcium | 0,09 |
| Benzoate de sodium | 0,10 |
| Smectite activée | 1,00 |
| | 7,35 |

Les compositions selon l'invention permettent de soulager les patients des symptômes de diarrhée tout en leur fournissant un apport compensatoire d'électrolyte, de sucre et de liquide. Bien que l'administration de l'électrolyte, du sucre, et de la substance absorbante soit conjointe, il n'a pas été constaté d'absorbtion de l'électrolyte et du sucre par cette substance, qui apporte une diminution notable de l'action des éléments de complément. Au contraire, on a pu constater que cette administration conjointe permet d'enraver beaucoup mieux la diarrhée que la seule administration d'électrolyte et de sucre ou que l'administration séquentielle de l'agent absorbant et des éléments de complément.

On a effectivement vérifié que le traitement d'enfants par administration séquentielle des deux types de produits conduisait à des résultats inférieurs à ceux obtenus par la composition selon l'invention, mais comparables à l'administration des seuls éléments de complément.

Expérimentation clinique :

100 enfants âgés de 1 à 24 mois et hospitalisés pour diarrhées aigües réclamant l'administration d'une solution orale de réhydratation ("SOR") ont été répartis en groupes de 50, équilibrés sur le plan de l'âge et de la sévérité de la symptomatologie.

Les enfants du groupe 1 ont été traités uniquement par la SOR. Les sels de réhydratation ont été présentés sous la forme de doses destinées à être dissoutes dans un litre d'eau de boisson et présentaient les caractéristiques suivantes :

Quantité

| Constituants | Quantité en g/l |
|---|---|
| Nacl | 3,5 |
| $CO_3HNa$ | 2,5 |
| KCl | 1,5 |
| Glucose | 20,0 |

Molarité

| Constituants | mmol/l en eau |
|---|---|
| Na | 90 |
| K | 20 |
| Cl | 80 |
| $CO_3H$ | 30 |
| Glucose | 111 |

Les enfants du groupe 2 ont reçu la même quantité de SOR mais incluse dans la composition selon l'invention sous la forme de 1 à 2 doses par jour contenant 3 g d'attapulgite activée. Les doses réellement administrées variaient en fonction du poids corporel comme indiqué plus haut.

6 critères de réalisation du traitement ont été retenus :

5

1. Nombre de selles après 48 heures de traitement,
2. Nombre de selles par 24 heures après 2 jours de traitement,
3. Gain pondéral quotidien,
4. Durée du traitement,
5. Fréquence des rechutes.

Caractéristiques des enfants traités

– Répartition par sexe:
  filles: 51
  garçons: 49
– Répartition par âge:
  2 à 12 mois: 51
  13 à 24 mois: 49
– Symptomatologie complémentaire:
  fièvre: 26
  vomissements: 48

Le nombre de selles par jour pour chacun des groupes avant traitement est donné dans le tableau suivant :

| Nombre de selles Groupe | 3 à 6 | 7 à 10 |
|---|---|---|
| SOR | 34 (68 %) | 16 (32 %) |
| SOR + ATTA activée | 44 (88 %) | 6 (12 %) |

Les posologies suivantes ont été retenues :
SOR : 1/4 à 1/2 litre par jour pour les enfants de moins de 6 mois ;
1/2 à 1 litre par jour pour les enfants de 6 à 24 mois.
Attapulgite : 1 dose par jour pour les enfants de moins de 5 kg.
2 doses par jour pour les enfants de plus de 5 kg.
Les résultats sont donnés en fonction des critères d'évaluation définis ci-dessus.

Résultats

1) Caractéristiques des selles après 48 heures de traitement

La classification a réparti les selles en 4 catégories : très liquides ; semi-liquides ; molles ; normales.

| | SOR (n = 50) | | SOR + ATTA (n = 50) | |
|---|---|---|---|---|
| | No. | % | No. | % |
| selles liquides | 26 | 52 | 10 | 20 |
| selles semi-liquides | 10 | 20 | 14 | 28 |
| selles molles | 11 | 22 | 18 | 36 |
| selles normales | 3 | 6 | 8 | 16 |

2) Nombre de selles par jour après 48 heures de traitement

Les valeurs sont données dans le tableau ci-dessous.

| Produit | Nombre de selles/jour à l'admission | après 48 heures de traitement |
|---|---|---|
| SOR | 6 | 4 |
| SOR + ATTA activée | 6 | 3 |

Après 48 heures de traitement 26 enfants, soit 52 % du groupe traité par la SOR présentaient encore des selles très liquides cependant cette forme de diarrhée n'impliquait plus de pronostic fatal pour ces enfants.

Dans le groupe traité par la composition selon l'invention, seulement 10 enfants, soit 20 %, présentaient des selles liquides. Toutefois la normalisation complète des selles a été notée dans 3 cas dans le groupe SOR contre 8 cas, soit 16 % avec le traitement selon l'invention. En même temps, on a noté une grande réduction de la fréquence des selles dans le groupe recevant le traitement combiné (3 par jour au lieu de 6 initialement), le chiffre, pour les enfants traités par la SOR, étant de 4 par jour au lieu de 6 initialement.

On peut donc conclure que la réduction de la perte en eau et en électrolyte était plus favorable dans le traitement selon l'invention que le traitement par la seule SOR.

3) Evolution des courbes de poids corporel sur 48 heures

Ces valeurs sont données dans le tableau suivant.

|  | 0 | +50-100g /jour | +100-150g /jour | +150-250g /jour | +250-350g /jour | LOSS |
|---|---|---|---|---|---|---|
| SOR | 0 | 35 | 14 | 0 | 0 | 1 |
| SOR + ATTA activée | 2 | 3 | 18 | 25 | 2 | 0 |

On a noté un gain de poids régulier pour 49 des enfants du groupe SOR et 48 du groupe SOR + attapulgite activée. Dans ce premier groupe, seul un enfant a présenté un signe d'aggravation de diarrhée après 48 heures avec une perte quotidienne de 50 g.

Pour les autres enfants du groupe SOR, le gain de poids a été régulier et progressif avec une valeur moyenne de 100 à 200 g et une rapide amélioration de l'état général.

Dans le groupe ayant reçu le traitement selon l'invention, la diarrhée devint plus aigüe pour 2 enfants mais néanmoins sans perte de poids alors que tous les autres enfants présentèrent des gains de poids quotidiens de 150 à 200 g et, dans certains cas, 300 g et plus.

On peut donc conclure que la prise de poids des 2 groupes a été satisfaisante.

4) Durée du traitement

Celui-ci été compté du début du traitement jusqu'à la normalisation de la symptomatologie digestive. Les résultats sont donnés dans le tableau ci-dessous.

| Nombre de jours de traitement | SOR | | SOR + ATTA activée | |
|---|---|---|---|---|
| 3 jours | 3 | 6 % | 8 | 16 % |
| 4 jours | 6 | 12 % | 38 | 76 % |
| 5 jours | 13 | 26 % | 4 | 8 % |
| 6 jours | 28 | 56 % | 0 | — |

Dans le cas du traitement de la SOR seule, la durée moyenne a été de 5 à 6 jours tandis que pour le traitement selon l'invention, elle a été plutôt de 3 à 4 jours.

5) Rechutes

Lorsque la diarrhée réapparaissait 3 jours après le retour à la normalité, ceci était considéré comme une rechute. Dans les expérimentations précédentes, il y a eu 7 cas de rechute dans le groupe traité par la SOR, 2 cas dans le groupe traité par la SOR + ATTA, et aucun dans le groupe SOR + smectite activée.

Une expérimentation a également été effectuée avec la composition 5 (smectite activée) sur deux groupes de 10 patients avec des résultats comparables à ceux obtenus précédemment.

Les valeurs correspondantes sont données dans les tableaux qui suivent.

Caractéristiques des selles après 48 heures de traitement

|  | SOR (n = 50) | | SOR + smectite activée (n = 50) | |
|---|---|---|---|---|
|  | No. | % | No. | % |
| selles liquides | 5 | 50 | 2 | 20 |
| selles semi-liquides | 2 | 20 | 3 | 30 |
| selles molles | 2 | 20 | 4 | 40 |
| selles normales | 1 | 10 | 1 | 10 |

Nombre de selles par jour après 48 heures de traitement

| Produit | Nombre de selles/jour | |
|---|---|---|
|  | à l'admission | après 48 heures de traitement |
| SOR | 6 | 4 |
| SOR + smectite activée | 6 | 3 |

Evolution des courbes de poids corporel sur 48 heures

|  | 0 | +50-100g /jour | +100-150g /jour | +150-250g /jour | +250-350g /jour |
|---|---|---|---|---|---|
| SOR | 1 | 7 | 2 | 0 | 0 |
| SOR + smectite activée | 0 | 1 | 3 | 5 | 1 |

**Revendications**

1. Composition thérapeutique à action anti-diarrhéique, contenant des quantités suffisantes pour réduire la diarrhée d'une argile à base de silicate double d'aluminium et de magnésium, thermiquement activée et finement pulvérisée, appartenant au groupe des attapulgites ou au groupe des smectites, comme substance susceptible d'absorber les bactéries intestinales pathogènes, d'un sel de sodium, d'un sel de potassium et de sucre.

2. Composition selon la revendication 1, dans laquelle le sucre est du glucose ou du dextrose.

3. Composition selon l'une des revendications 1 ou 2, contenant, en outre, du bicarbonate de sodium.

4. Composition selon l'une des revendications précédentes, comprenant, en outre, un agent de mise en suspension.

5. Composition selon la revendication 4, dans laquelle l'agent de mise en suspension est un hydrocolloide, un polyalcool ou un surfactif.

6. Composition selon l'une des revendications précédentes, dispersée, mise en suspension ou dissoute dans de l'eau et contenant, par litre, de 2,5 à 15 g de la substance absorbante, de 10 à 30 g de glucose, de 60 à 120 mEq de sodium, de 10 à 50 mEq de bicarbonate et de 10 à 30 mEq de potassium.

7. Composition selon l'une des revendications 1 à 5, se présentant sous la forme d'un mélange solide destiné à être additionné d'eau avant l'administration au malade.

8. Composition selon la revendication 7, conditionnée sous la forme d'unités de dosage, les quantités d'ingrédient conditionné étant telles que, lors de la dispersion, de la mise en suspension ou de la dissolution de ceux-ci dans 200 ml d'eau, elles conduisent à une composition conforme à celle de la revendication 6.

**Claims**

1. An anti-diarrhea composition containing diarrhea symptom-reducing amounts of a thermally activated, finely powdered, hydrous magnesium aluminium silicate clay belonging to the group of the attapulgites or to the group of the smectites as absorptive material capable of absorbing pathogenic intestinal bacteria, a sodium salt, a potassium salt and a sugar.

2. A composition according to claim 1 in which the sugar is glucose or dextrose.

3. A composition according to claim 1 or claim 2 which contains sodium bicarbonate.

4. A composition according to any preceding claim further comprising a suspending agent.

5. A composition according to claim 4 in which the suspending agent is a hydrocolloid, a polyol or a surfactant.

6. A composition according to any preceding claim, the composition being dispersed, suspended or dissolved in water and containing, per litre, from 2.5 to 15 g of the absorptive material, from 10 to 30 g of glucose, from 60 to 120 mEq of sodium, from 10 to 50 mEq of bicarbonate and from 10 to 30 mEq of potassium.

7. A composition according to any of claims 1 to 5, the composition being in solid form for admixture with water prior to administration to a patient.

8. A composition according to claim 7 packaged in unit dosage form, the quantities of packaged material being such that their dispersion, suspension or dissolution in 200 ml of water gives a composition according to claim 6.

**Patentansprüche**

1. Therapeutische Zusammensetzung mit Anti-Diarrhöe-Wirkung, welche zur Verminderung der Diarrhöe ausreichende Mengen eines thermisch aktivierten und fein pulverisierten Tons auf Basis eines Doppelsilikats von Aluminium und Magnesium, welcher der Gruppe der Attapulgite oder der Gruppe der Smektite angehört, als zur Absorption von pathogenen Darmbakterien befähigte Substanz, ein Natriumsalz, ein Kaliumsalz und Zucker enthält.·

2. Zusammensetzung nach Anspruch 1, worin der Zucker Glukose oder Dextrose ist.

3. Zusammensetzung nach einem der Ansprüche 1 oder 2, welche außerdem Natriumbicarbonat enthält.

4. Zusammensetzung nach einem der vorstehenden Ansprüche, welche außerdem ein Suspensionshilfsmittel enthält.

5. Zusammensetzung nach Anspruch 4, worin das Suspensionshilfsmittel ein Hydrokolloid, ein Polyalkohol oder ein oberflächenaktives Mittel ist.

6. Zusammensetzung nach einem der vorstehenden Ansprüche, welche in Wasser dispergiert, suspendiert oder gelöst ist und, pro Liter, 2,5 bis 15 g der absorbierenden Substanz, 10 bis 30 g Glukose, 60 bis 120 mEq Natrium, 10 bis 50 mEq Bicarbonat und 10 bis 30 mEq Kalium enthält.

7. Zusammensetzung nach einem der Ansprüche 1 bis 5, welche in der Form einer festen Mischung vorliegt, die, vor der Verabreichung an den Kranken, zur Zugabe zu Wasser bestimmt ist.

8. Zusammensetzung nach Anspruch 7, konditioniert in Form von Einheitsdosen, wobei die Mengen an konditioniertem Bestandteil so sind, daß sie nach ihrer Dispersion, Suspendierung oder Auflösung in 200 ml Wasser zu einer Zusammensetzung führen, die mit der des Anspruchs 6 übereinstimmt.